# EUROPÄISCHE PATENTSCHRIFT

(11) **EP 1 135 076 B1**
(45) Veröffentlichungstag und Bekanntmachung des Hinweises auf die Patenterteilung: **23.11.2005**
(21) Anmeldenummer: 99964510.4
(22) Anmeldetag: 26.11.1999
(51) Int. Cl.: A61B 17/70

(54) **DISTRAKTIONSVORRICHTUNG**
DISTRACTION DEVICE
DISPOSITIF DE TRACTION

(30) Priorität: 04.12.1998 DE 19856013
(43) Veröffentlichungstag der Anmeldung: 26.09.2001
(73) Patentinhaber: Wittenstein AG, 97999 Igersheim (DE)
(72) Erfinder: KLEIN, Jürgen, D-97990 Weikersheim (DE); STAUCH, Roman, D-97959 Assamstadt (DE)
(74) Vertreter: Weiss, Peter
(86) Internationale Anmeldenummer: PCT/EP1999/009191
(87) Internationale Veröffentlichungsnummer: WO 2000/033752

(56) Entgegenhaltungen:
- EP-A- 0 820 731
- WO-A-98/47438
- DE-U- 9 107 494
- US-A- 5 575 790
- US-A- 5 626 579
- PATENT ABSTRACTS OF JAPAN vol. 1997, no. 07, 31. Juli 1997 (1997-07-31) & JP 09 056736 A (TANAKA IKA) & DATABASE WPI Section PQ, Week 199719 Derwent Publications Ltd., London, GB; Class P31, AN 1997-206966 XP002135549

## Beschreibung

Die vorliegende Erfindung betrifft eine Distraktionsvorrichtung zur operativen Korrektur von Wirbelsäulenerkrankungen, Schädigungen oder Missbildungen, wie Wirbelsäulenverkrümmungen, Spina bifida, die Spondylolisthesis, Spondylarthrosis, mit einer Einrichtung aus wenigstens zwei axial gegeneinander bewegbaren Elementen, welche an benachbarten Wirbeln oder wirbelbereichen ggf. beabstandet angreifen, wobei die Einrichtung subkutan, vollimplantiert einsetzbar und berührungslos von aussen betätigbar ist, und wobei die Elemente der Einrichtung mittels Formgedächtnisaktuatoren gegeneinander bewegbar sind.

Mit herkömmlichen Distraktionsvorrichtungen werden Knochen, Wirbel etc. durch Anbringen von Distraktionsstäben und Erzeugung einer Distraktionskraft gestreckt. Eine entsprechende Dehnung oder Distraktion kann durch eine Verlängerung der Distraktionsstäbe hervorgerufen werden.

Nachteilig an derartigen bisherigen Distraktionsvorrichtungen ist, dass eine Korrektur nur einmal in einer Sitzung bzw. operativen Behandlung möglich ist. Zudem ist die Krafteinleitung des Distraktionsstabes nur über zwei wirbel möglich. Die Distraktionskraft, welche von Wirbelbögen maximal aufgenommen werden kann, beläuft sich auf etwa 30 bis 40 Kilopond. Bei grösseren Distraktionskräften besteht die Gefahr des Ausbrechens an den distalen Wirbelbögen.

Problematisch ist ferner, dass eine Distraktion immer nur vor Ort durch einen operativen Eingriff vorgenommen werden kann. Häufig sind jedoch Korrekturen, insbesondere auch bei Kindern, die sich noch im Wachstum befinden, erforderlich. Diese Korrekturen würden eine Nachoperation erfordern, was unerwünscht ist.

Ferner sind Korrekturen nur langsam vorzunehmen, da die Geweberelaxation bzw. der langsame biologische Adaptionsprozess dies erfordert.

Zudem stellt sich die Problematik bei der operativen Skoliosekorrektur, dass häufig Infektionsherde durch das Einleiten und durch häufiges Nachoperieren entstehen.

Nach der US 5,626,579 ist ein implantierbares chirurgisches Instrument aufgezeigt, welches über einen mechanisch betätigten Aktuator eine Distraktion zweier Elemente zulässt. Über entsprechende Magneten lässt sich der magnetische Aktuator verdrehen, um über eine Trommel ein Zugseil zum Auseinanderbewegen der Elemente zu gewährleisten.

Die wo 98/47 438 beschreibt eine Knochendistraktionsvorrichtung, bei welcher ein Druckgeber anschliesst, der ggf. energiebeaufschlagbar ist. Nachteilig ist an einem derartigen Druckgeber, dass dieser sehr viel Raum unter der Haut einnimmt und den Patienten behindert.

Die EP 0 820 731 A1 beschreibt eine pneumatische Distraktionsvorrichtung bei welcher Hydraulikzylinder verwendet werden.

Die US 5,575,790 beschreibt einen mittels Formgedächntislegierungen betriebenen Aktuator, welchem von aussen zum Betreiben der Formgedächtnislegierungen zusätzlich Wärme zugeführt wird.

Das Deutsche Gebrauchsmuster 91 07 494.0 offenbart einen Wirbelkörperersatz rein mechanischer Art, der zwischen zwei Wirbeln eingesetzt wird.

Der Patent Abstracts of Japan vol. 1997, Nr. 07, vom 31.07.1997 sowie die JP 09 056736 A offenbaren eine rein mechanische Knochendistraktionsvorrichtung zum Verlängern von Knochen. Dabei wird über einen Elektromotor eine Spinndel zur Distraktion angetrieben.

Der vorliegenden Erfindung liegt die Aufgabe zugrunde, eine Distraktionsvorrichtung zu schaffen, welche die genannten Nachteile beseitigt und mit welcher, insbesondere ein Ausreissen von Verankerungen an den Wirbeln oder deren Bestandteile vermieden wird. Es sollen ferner grössere Korrekturbereiche bei geringerer humaner Belastung und insbesondere auch Systembelastung möglich sein.

Zur Lösung dieser Aufgabe führt, dass die Einrichtung über eine Verbindungsleitung mit einem Energieübertragungselement verbunden ist, an welches berührungslos elektrische Energie von aussen induktiv zum definierten Auseinanderbewegen der Elemente zuführbar ist, und dass das Energieübertragungselement über die Verbindungsleitung subkutan ausserhalb eines Bereiches der Wirbelsäule implantierbar ist.

Bei der vorliegenden Erfindung ist von Bedeutung, dass subkutan die Einrichtung mittelbar oder unmittelbar betrieben, berührungslos von aussen, betätigt werden kann. Dies geschieht mittels Induktion, in dem ein Energieübertragungselement die Einrichtung induktiv von aussen in Betrieb setzt. Dabei kann die Distraktionseinrichtung zwischen zwei wirbeln eingesetzt sein, wenn beispielsweise eine entsprechende Ausnehmung durch eine Wirbelbehinderung vorhanden ist. Das Energieübertragungselement kann aus einem Sendekopf, dem Transmitter und dem sukutan implantierten Empfänger, dem Receiver bestehen.

Die Einrichtung weist zwei axial gegeneinander bewegbare Elemente auf, die als auseinanderfahrbare Zylinder ausgebildet sind. Da zwischen zwei Wirbeln sehr wenig Raum ist, kann eine entsprechende Einrichtung nur dann eingesetzt werden, wenn beispielsweise ein Wirbel durch Miss- oder Fehlbildung oder sogar Krankheit fehlt.

Damit die Korrektur permanent vorgenommen werden kann, lässt sich die Distraktionsvorrichtung vollständig subkutan implantieren. Dabei schliesst an die Einrichtung eine Verbindungsleitung zum Energieübertragungselement an. Dieses Energieübertragungselement kann in der Nähe oder äuch in ganz anderen Körperbereichen unter der Haut sitzen, um die Einrichtung in Betrieb zu setzen.

Damit die Distraktionsvorrichtung zwischen zwei Wirbeln beweglich eingesetzt werden kann, schliessen endseits an die zylinderartigen Elemente der Einrichtung Plattenelemente an, welche gelenkartig über ein Gelenk mit diesem in Verbindung stehen. Nach aussen gerichtete Rastelemente, Zacken od. dgl. verhindern ein Abrutschen der Distraktionsvorrichtung und positionieren diese exakt am Wirbel. Durch diese Gelenke ist eine Bewegung der Wirbelsäule uneingeschränkt möglich.

Ferner ist von Vorteil, dass bei der vollständigen subkutanen implantierten Distraktionsvorrichtung eine Distraktion, beispielsweise unter Röntgen kontrolliert und nachgestellt werden kann. Die Korrekturen können auf diese Weise überprüft werden, so dass die Wirbelsäule ihre gewünschte Form erhält.

In einem weiteren Ausführungsbeispiel ist eine Distraktionsvorrichtung zur seitlichen Korrektur der Wirbelsäule, insbesondere im Lendenwirbelbereich eingesetzt, wobei endseits der ausfahrbaren Elemente der Einrichtung Hakenelemente verstellbar vorgesehen sind. Diese Hakenelemente sind nach aussen geöffnet und können jeweilse den Processus costalis umgreifen. Durch die Distraktion in oben beschriebener Weise lässt sich eine seitliche Krümmung der Wirbelsäule ausgleichen. Auch ein Nachjustieren und ein Korrigieren ist hier möglich, da subkutan das Energieübertragungselement zum Einspeisen von Energie, induktiv und berührungslos von aussen, zum Steuern und Ausfahren der Distraktionsvorrichtung vorgesehen ist.

Insgesamt bietet die vorliegende Erfindung langfristige Möglichkeiten Distraktionsvorrichtungen ohne zusätzliche operative Eingriffe zu betreiben. Dies ist insbesondere dann von Vorteil, wenn häufig Korrekturen, beispielsweise bei Kindern durch Wachstum, erforderlich sind.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine schematisch dargestellte Draufsicht einer erfindungsgemässen Distraktionsvorrichtung mit subkutan anschliessendem Energieübertragungselement in Gebrauchslage;
Figur 2 eine schematisch dargestellte Draufsicht eines weiteren Ausführungsbeispieles einer Distraktionsvorrichtung mit anschliessendem Energieübertragungselement.

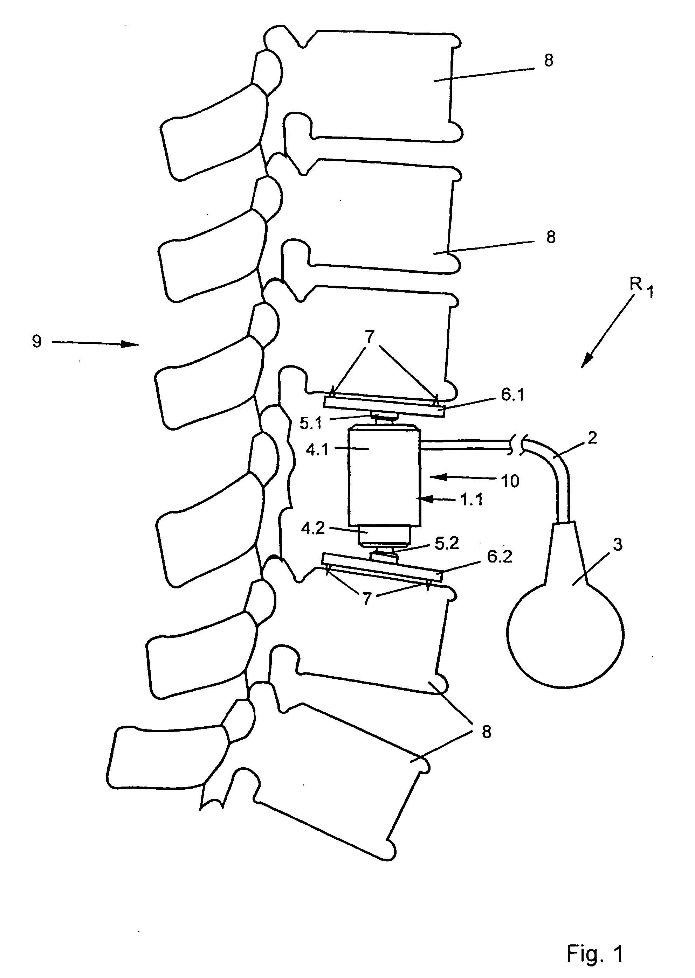

Gemäss Figur 1 weist eine erfindungsgemässe Distraktionsvorrichtung R₁ eine Einrichtung 1.1 auf, an welche über eine Verbindungsleitung 2 ein Energieübertragungselement 3 anschliesst. Die Verbindungsleitung 2 kann von beliebiger und wählbarer Länge sein. Die Einrichtung 1.1 weist ferner zwei axial gegeneinander bewegbare Elemente 4.1, 4.2, insbesondere Zylinder auf, welche über Aktuatoren aus Formgedächtnislegierungen die Elemente 4.1, 4.2 gegeneinander bewegbar sind.

Endseits an den Elementen 4.1, 4.2 schliessen über Gelenke 5.1, 5.2 Plattenelemente 6.1, 6.2 an. Nach aussen ragen von den Plattenelementen 6.1, 6.2 eine Mehrzahl von Rastelementen 7 ab, welche ein Abrutschen an einem Wirbel 8 einer Wirbelsäule 9 verhindern.

Im vorliegenden Ausführungsbeispiel ist die Distraktionsvorrichtung R₁, insbesondere deren Einrichtung 1.1 zwischen zwei Wirbeln 8 der Wirbelsäule 9 in eine Ausnehmung 10 eingesetzt. Die Ausnehmung 10 kann beispielsweise durch eine Missbildung eines Wirbels, eine Beschädigung, Erkrankung oder sogar durch einen Tumor erfolgt sein. Hierdurch lässt sich durch Distraktion diese Beschädigung überbrücken, so dass beispielsweise ein Wachstumsverhalten der Wirbelsäule 9 ausgeglichen werden kann.

Wichtig bei der vorliegenden Erfindung ist jedoch, dass über die Verbindungsleitung 2 das subkutane vollimplantierbare Energieübertragungselement 3 ausserhalb des Wirbelsäulenbereiches unter der Haut einsetzbar ist. Dies minimiert das Infektionsrisiko erheblich und gewährleistet einen enormen Behandlungskomfort. Für den Patienten ist eine derartige Behandlung, insbesondere eine Distraktion keine Einschränkung und Belastung.

Mittels des Energieübertragungselementes 3 lassen sich die Elemente 4.1, 4.2, insbesondere die Zylinder durch induktive Energiezufuhr betreiben und auseinanderbewegen, um eine Distraktion, wenn diese gewünscht wird, in beliebigen Zeitabständen zu erzeugen.

Dabei kann von Zeit zu Zeit, beispielsweise im Wachstum, bei Krümmungsschäden ein Nachstellen auf einfache Weise erfolgen.

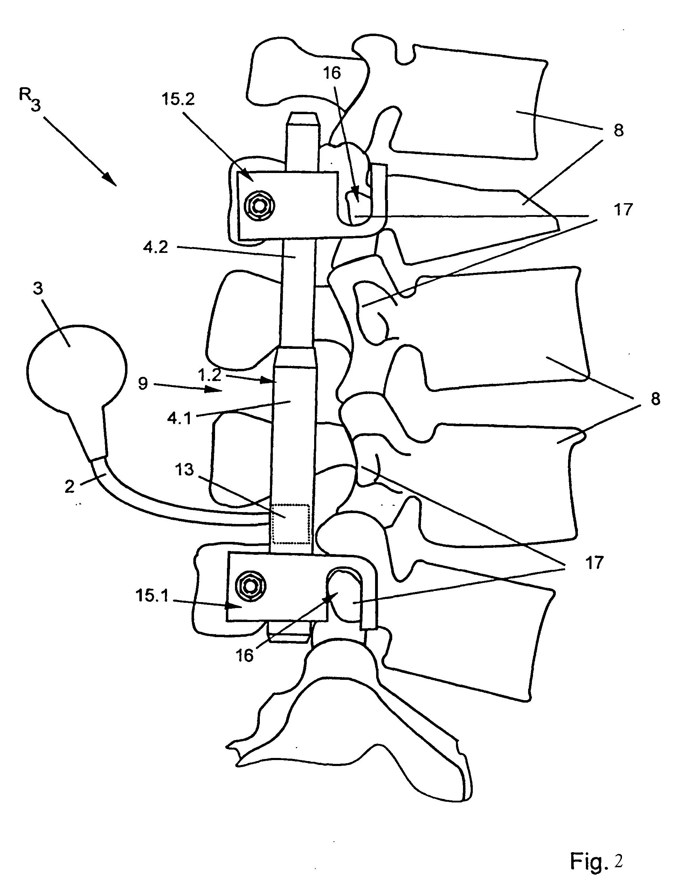

In Figur 2 ist eine weitere Distraktionsvorrichtung R₃ aufgezeigt, bei welcher seitlich die Lendenwirbel dargestellt sind. Seitliche Krümmungen der Wirbelsäule 9, beispielsweise durch Missbildungen, Erkrankungen der Wirbel, insbesondere der Lendenwirbel können mittels der erfindungsgemässen Distraktionsvorrichtung R₃ durch die Distraktion ausgeglichen werden. Hierzu sitzt die Distraktionsvorrichtung R₃ seitlich an der Wirbelsäule 9. Diese kann ein- oder beidseitig an der Wirbelsäule angeordnet sein.

Gerade auch durch Veränderungen der Wirbelsäule 9 auch im Wachstum ist es erforderlich, dass eine Distraktion von Zeit zu Zeit zu erfolgen hat, um wachstumsbedingte Änderungen ohne Operationen zu korrigieren.

Die entsprechende Einrichtung 1.2 weist ebenfalls ein erstes Element 4.1 und ein gegenüber diesem axial bewegbaren zweiten Element 4.2 auf. Vorzugsweise sind diese auch als ineinander verschiebbare Zylinder ausgebildet. Endseits der Elemente 4.1, 4.2 sind Hakenelemente 15.1, 15.2 vorgesehen, welche mit einer Öffnung 16 den seitlich abragenden Processus costalis 17 umfangen. Dabei sind die Öffnungen 16 der Hakenelemente 15.1, 15.2 nach aussen gerichtet, um den Processus costalis 17 dort aufzunehmen und beim Auseinanderbewegen der Elemente 4.1, 4.2 eine Distraktion zu bewirken. Die Distraktion erfolgt, wie oben beschrieben, über ein entsprechendes Antriebsmittel 13, welches über die Verbindungsleitung 2 von dem Energieübertragungselement 3 subkutan mit Energie versorgt wird. Auch hier ist daran gedacht, die Distraktionsvorrichtung R₃ sehr klein auszubilden, so dass eine entsprechende Betätigungseinrichtung 11 subkutan zwischen Energieübertragungselement 3 und Einrichtung 1.2 zwischengeschaltet werden kann.

Die Hakenelemente 15.1, 15.2 sind auf den Elementen 4.1, 4.2 axial verschiebbar gelagert. Diese können über entsprechende Klemmverbindungen voreingestellt werden, so dass ein gewünschter Abstand zu jedem beliebigen Wirbel voreingestellt werden kann. Auf diese Weise lässt sich die Distraktionsvorrichtung R₃ auf vielfältigste Weise einsetzen, wobei auch unterschiedlich grosse Bereiche mit mehreren dazwischenliegenden Wirbeln gestreckt werden können.

## Patentansprüche

1. Distraktionsvorrichtung zur operativen Korrektur von Wirbelsäulenerkrankungen, Schädigungen oder Missbildungen, wie Wirbelsäulenverkrümmungen, Spina bifida, die Spondylolisthesis, Spondylarthrosis, mit einer Einrichtung (1) aus wenigstens zwei axial gegeneinander bewegbaren Elementen (4.1, 4.2), welche an benachbarten Wirbeln oder Wirbelbereichen beabstandet angreifen, wobei die Einrichtung (1) subkutan, vollimplantiert einsetzbar und berührungslos von aussen betätigbar ist, und wobei die Elemente (4.1, 4.2) der Einrichtung (1) mittels Formgedächtnisaktuatoren gegeneinander bewegbar sind,
**dadurch gekennzeichnet,**
**dass** die Einrichtung (1) über eine Verbindungsleitung (2) mit einem Energieübertragungselement (3) verbunden ist, an welches berührungslos elektrische Energie von aussen induktiv zum definierten Auseinanderbewegen der Elemente (4.1, 4.2) zuführbar ist, und dass das Energieübertragungselement (3) über die Verbindungsleitung (2) subkutan ausserhalb eines Bereiches der Wirbelsäule (9) implantierbar ist.

2. Distraktionsvorrichtung nach Anspruch 1, **dadurch gekennzeichnet, dass** die Elemente (4.1, 4.2) der Einrichtung (1) zylinderartig ausgebildet und ineinander verfahrbar sind.

3. Distraktionsvorrichtung nach Anspruch 2, **dadurch gekennzeichnet, dass** endseits an die Elemente (4.1, 4.2) Plattenelemente (6.1, 6.2) anschliessen.

4. Distraktionsvorrichtung nach Anspruch 3, **dadurch gekennzeichnet, dass** zwischen Plattenelementen (6.1, 6.2) und Elementen (4.1, 4.2) ein Gelenk (5.1, 5.2) vorgesehen ist.

5. Distraktionsvorrichtung nach Anspruch 3 oder 4, **dadurch gekennzeichnet, dass** die Plattenelemente (6.1, 6.2) mit wenigstens einem Rastelement (7) versehen sind.

6. Distraktionsvorrichtung nach wenigstens einem der Ansprüche 1 bis 5, **dadurch gekennzeichnet, dass** die Einrichtung (1) mittels endseits vorgesehenen Hakenlementen (15.1, 15.2) in jeweils einen Processus costalis (17) eines Wirbel (8) eingreifen kann und durch ein Auseinanderbewegen der Elemente (4.1, 4.2) eine Distraktion dieses Abschnittes erfolgen kann.

7. Distraktionsvorrichtung nach Anspruch 6, **dadurch gekennzeichnet, dass** das Hakenelement (15.1, 15.2) mit einer Öffnung (16) versehen ist, in welche der Processus costalis (17) eingreifen kann.

8. Distraktionsvorrichtung nach Anspruch 6 oder 7, **dadurch gekennzeichnet, dass** die Halteelemente (15.1, 15.2) endseits an den Elementen (4.1, 4.2) wiederlösbar vorgesehen sind.

9. Distraktionsvorrichtung nach wenigstens einem der Ansprüche 6 bis 8, **dadurch gekennzeichnet, dass** die Hakenelemente (15.1, 15.2) axial auf den Elementen (4.1, 4.2) verschiebbar und verstellbar angeordnet sind.

## Claims

1. Distraction apparatus for surgically correcting spinal disorders, damage or deformities, such as curvature of the spine, spina bifida, spondylolisthesis or spondylarthrosis, said apparatus having an arrangement (1) formed from at least two elements (4.1, 4.2), which are axially displaceable relative to each other and act on adjacent vertebrae or vertebral regions with a spacing therebetween, the arrangement (1) being subcutaneous, being insertable in a fully implanted manner and being contactlessly actuatable from externally, and the elements (4.1, 4.2) of the arrangement (1) being displaceable relative to each other by means of shape-memorising actuators, **characterised in that** the arrangement (1) is connected via a connecting line (2) to an energy transmitting element (3), to which electrical energy is suppliable contactlessly from externally in an inductive manner for the specific moving apart of the elements (4.1, 4.2), and **in that** the energy transmitting element (3) can be implanted subcutaneously externally of a region of the spine (9) via the connecting line (2).

2. Distraction apparatus according to claim 1, **characterised in that** the elements (4.1, 4.2) of the arrangement (1) have a cylindrical configuration and are displaceable one inside the other.

3. Distraction apparatus according to claim 2, **characterised in that** plate elements (6.1, 6.2) fit terminally on the elements (4.1, 4.2).

4. Distraction apparatus according to claim 3, **characterised in that** a pivot joint (5.1, 5.2) is provided between plate elements (6.1, 6.2) and elements (4.1, 4.2).

5. Distraction apparatus according to claim 3 or 4, **characterised in that** the plate elements (6.1, 6.2) are provided with at least one locking element (7).

6. Distraction apparatus according to at least one of claims 1 to 5, **characterised in that** the arrangement (1) can engage in a respective Processus costalis (17) of a vertebra by means of terminally provided hook elements (15.1, 15.2), and a distraction of this portion can be effected by moving the elements (4.1, 4.2) apart.

7. Distraction apparatus according to claim 6, **characterised in that** the hook element (15.1, 15.2) is provided with an opening (16), in which the Processus costalis (17) can engage.

8. Distraction apparatus according to claim 6 or 7, **characterised in that** the hook elements (15.1, 15.2) are re-releasably provided terminally on the elements (4.1, 4.2).

9. Distraction apparatus according to at least one of claims 6 to 8, **characterised in that** the hook elements (15.1, 15.2) are disposed so as to be displaceable and adjustable axially on the elements (4.1, 4.2).

## Revendications

1. Dispositif de distraction destiné à la correction opérative de maladies de la colonne vertébrale, de lésions ou déformations, telles que déformations de la colonne vertébrale, spina bifida, spondylolisthésis, spondylarthrose, avec un dispositif (1) composé d'au moins deux éléments (4.1, 4.2) déplaçables axialement l'un par rapport à l'autre qui viennent en prise, distants l'un de l'autre, avec des vertèbres ou zones de vertèbre adjacentes, le dispositif (1) pouvant être utilisé de manière sous-cutanée, entièrement implantée, et être actionné sans contact de l'extérieur, et les éléments (4.1, 4.2) du dispositif (1) pouvant être déplacés l'un par rapport à l'autre au moyen d'actionneurs à mémoire de forme.
**caractérisé par le fait que**
le dispositif (1) est relié, par l'intermédiaire d'une ligne de connexion (2), à un élément de transmission d'énergie (3), auquel de l'énergie électrique peut être alimentée sans contact, inductivement, de l'extérieur en vue d'un écartement défini des éléments (4.1, 4.2) l'un de l'autre, et que l'élément de transmission d'énergie (3) peut être implanté, par l'intermédiaire de la ligne de connexion (2), de manière sous-cutanée en dehors d'une zone de la colonne vertébrale.

2. Dispositif de distraction selon la revendication 1, **caractérisé par le fait que** les éléments (4.1, 4.2) du dispositif (1) sont réalisés en forme de cylindre et de manière déplaçables l'un dans l'autre.

3. Dispositif de distraction selon la revendication 2, **caractérisé par le fait que** du côté de l'extrémité aboutent, aux éléments (4.1, 4.2), des éléments de plaque {6.1, 6.2).

4. Dispositif de distraction selon la revendication 3, **caractérisé par le fait qu'**il est prévu, entre les éléments de plaque (6.1, 6.2) et les éléments (4.1, 4.2), une articulation (5.1, 5.2).

5. Dispositif de distraction selon la revendication 3 ou 4, **caractérisée par le fait que** les éléments de plaque (6.1, 6.2) sont pourvues d'au moins un élément d'encliquetage (7).

6. Dispositif de distraction selon au moins l'une des revendications 1 à 5, **caractérisée par le fait que** le dispositif (1) peut s'engager, au moyen de chacun des éléments de crochet (15.1, 15.2) prévus du côté de l'extrémité, dans un processus costal (17) d'une vertèbre (8) et que par un écartement des éléments (4.1, 4.2) l'un de l'autre peut avoir lieu une distraction de ce segment.

7. Dispositif de distraction selon la revendication 6, **caractérisé par le fait que** l'élément de crochet (15.1, 15.2) est pourvu d'une ouverture (16) dans laquelle peut s'engager le processus costal (17).

8. Dispositif de distraction selon la revendication 6 ou 7, **caractérisé par le fait que** les éléments de crochet (15.1, 15.2) sont prévus de manière amovible, du côté de l'extrémité, aux éléments (4.1, 4.2).

9. Dispositif de distraction selon au moins l'une des revendications 6 à 8, **caractérisé par le fait que** les éléments de crochet (15.1, 15.2) sont disposés de manière déplaçable axialement et réglable sur les éléments (4.1, 4.2).
